(19) Europäisches Patentamt
European Patent Office
Office européen des brevets

(11) Veröffentlichungsnummer: **0 259 248 B1**

(12) **EUROPÄISCHE PATENTSCHRIFT**

(45) Veröffentlichungstag der Patentschrift: **01.07.92**

(51) Int. Cl.$^5$: **C07J 71/00**

(21) Anmeldenummer: **87730099.6**

(22) Anmeldetag: **28.08.87**

# (54) 13alpha-Alkylgonan-delta9(11)-5,10-epoxide.

(30) Priorität: **01.09.86 DE 3630030**

(43) Veröffentlichungstag der Anmeldung:
**09.03.88 Patentblatt 88/10**

(45) Bekanntmachung des Hinweises auf die Patenterteilung:
**01.07.92 Patentblatt 92/27**

(84) Benannte Vertragsstaaten:
**AT BE CH DE ES FR GB GR IT LI LU NL SE**

(56) Entgegenhaltungen:
**EP-A- 245 170**
**EP-A- 0 129 499**
**FR-E- 95 149**

**TETRAHEDRON LETTERS, Band 26, Nr. 17, 1985, Seiten 2069-2072, Pergamon Press, Oxford, GB; R. ROHDE et al.: "Stereoselective epoxidation of 5(10), 9(11)-estradienes"**

(73) Patentinhaber: **SCHERING AKTIENGESELLSCHAFT Berlin und Bergkamen**
**Müllerstrasse 170/178 Postfach 65 03 11**
**W-1000 Berlin 65(DE)**

(72) Erfinder: **Neef, Günter, Dr.**
**Darmstädter Strasse 9**
**W-1000 Berlin 15(DE)**
Erfinder: **Vierhufe, Harry**
**Margaretenstrasse 37**
**W-1000 Berlin 45(DE)**

**EP 0 259 248 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

11β-Aryl-substituierte 13α-Alkylgonane sind als wirksame Antigestagene und Antiglucocorticoide beschrieben worden (Steroids 44,349 [1984], Europäische Patentanmeldung Publikations-Nr. 0129 499).

Ihre Herstellung erfolgt im bisherigen verfahren über ein 11β-Aryl-substituiertes 13α-Alkyl-5α-Hydroxy-17-oxo-gonan-Zwischenprodukt, das durch nukleop hile Addition einer Seitenkette an das 17-Keton, gegebenenfalls Variation des C-17-Substituentenmusters und anschließende Wasserabspaltung unter gleichzeitiger Abspaltung vorhandener Schutzgruppen in das jeweils gewünschte Endprodukt überführt wird. Ein gravierender Nachteil dieses Verfahrens ist die nicht-stereoselektiv verlaufende Einführung der C-17-Seitenkette in das Steroidgerüst. Dadurch wird eine ausbeutemindernde und meistens sehr aufwendige Trennung beider Epimeren notwendig.

Der Erfindung liegt somit die Aufgabe zugrunde, ein Zwischenprodukt, das nur das gewünschte Seitenketten-Isomere liefert, und ein Verfahren zu seiner Herstellung zu schaffen. Diese Aufgabe wird durch die Erfindung gelöst. Die Erfindung betrifft somit den in den Ansprüchen gekennzeichneten Gegenstand, d.h. neue 13α-Alkylgon-$\Delta^{9(11)}$-en-5,10-epoxide sowie Verfahren zu ihrer Herstellung.

Die erfindungsgemäßen Verbindungen werden durch die allgemeine Formel I

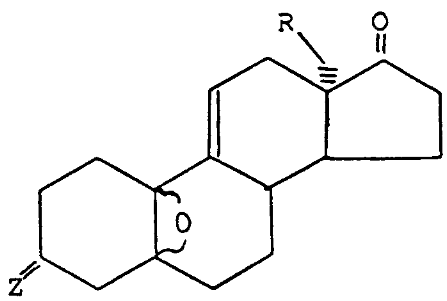

(I),

worin
R ein Wasserstoffatom, eine Methyl- oder Ethylgruppe und
Z eine in Form des Ketals oder des Thioketals blockierte Ketogruppe bedeuten,
beschrieben.

Die von Z umfaßten Schutzgruppen sind im sauren Milieu leicht abspaltbare Gruppen, wie z.B. die Ethylendioxyketal-, Ethylendithioketal-oder 2,2-Dimethyl-trimethylendioxyketalgruppe.

Die neuen 13α-Alkylgon-$\Delta^{9(11)}$-en-5,10-epoxide der allgemeinen Formel I werden erfindungsgemäß nach dem Verfahren gemäß Anspruch 2 hergestellt. Man geht dabei aus von einem z.B. nach Tetrahedron Letters 26, 2096 (1985) zugänglichen$\Delta^9$-Estren-Epoxid der allgemeinen Formel II

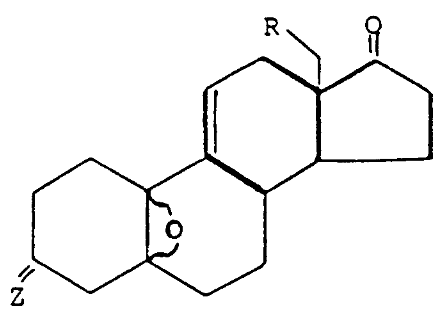

(II),

worin R und Z die in Formel I angegebene Bedeutung haben. Dieses Epoxid wird durch Bestrahlung mit ultraviolettem Licht in sein 13α-Epimeres überführt.

Die photochemische Umsetzung wird dabei vorzugsweise in einer Tauchapparatur oder mittels eines Fallfilm-Photoreaktors durchgeführt, wobei als Strahlungsquelle ein Quecksilberhochdruckbrenner Verwendung findet. Die Apparatur besteht aus Quarzglas oder einem Spezialglas geeigneter spektraler Durchlässigkeit. Bevorzugte Lösungsmittel sind unpolare aprotische Solventien wie Hexan, Cyclohexan, Benzol, Toluol, Tetrahydrofuran, Dioxan oder deren Gemische. Die Bestrahlungsdauer beträgt 1 bis 4 Stunden,

wobei ein Temperaturbereich von -30 bis +30 °C genutzt wird. Die Produkte der allgemeinen Formel I werden durch Kristallisation oder vorzugsweise durch Chromatographie gereinigt. Die Konzentration der zur Bestrahlung eingesetzten Lösungen beträgt 0,5 bis 3,0 Gew.-Prozent. Die erzielten Ausbeuten liegen im Bereich von 45 bis 60 %.

Durch das erfindungsgemäße Verfahren werden die neuen Zwischenprodukte der allgemeinen Formel I erhalten, die bei der nachfolgenden nukleophilen Addition der C-17-Seitenkette -wie an der Darstellung des beispielmäßig angeführten 3,3-(2,2-Dimethyl-trimethylendioxy)-5α,10α-epoxy-13α-methyl--17β-[3-(tetrahydropyran-2-yloxy)-prop-1-inyl]-9(11)-gonen-17α-ol gezeigt werden soll - ausschließlich das gewünschte α-Hydroxy-Epimere liefern:

Eine Lösung von 13,5 g Propargyl-THP-Ether in 160 ml abs. Tetrahydrofuran wird bei 0 °C tropfenweise mit 76,7 ml einer 1,6-molaren Lösung von n-Butyllithium in Hexan versetzt. Man rührt 15 Minuten bei 0 °C nach, kühlt dann auf -20 °C und tropft anschließend eine Lösung von 4,91 g des nach Beispiel 1 erhaltenen 3,3-(2,2-Dimethyl-trimethylendioxy)-5α,10α-epoxy--13α-methyl-gon-9(11)-en-17-on in 80 ml abs. THF hinzu. Nach Zugabe rührt man weitere 60 Minuten bei - 20 °C, gießt dann in ca. 2 l Wasser und extrahiert mit Ethylacetat. Nach Chromatographie des Rohproduktes an Aluminiumoxid (Merck, neutral, Stufe III) mit Hexan/Ethylacetat erhält man 5,48 g (81,1 %der Theorie) 3,3-(2,2-Dimethyl-trimethylendioxy)-5α,10α-epoxy-13α-methyl-17β-[3-(tetrahydropyran-2-yloxy)-prop-1-inyl]-9(11)--gonen-17α-ol als farbloses Ol, $^{1}$H-NMR ($CDCl_3$): δ = 0,86 ppm (s, 3H, 13α-$CH_3$); 0,98 (s, 3H, Ketal-$CH_3$); 1,05 (s, 3H,Ketal-$CH_3$); 4,32 (dd, 2H,C=C--$CH_2$OTHP); 4,82 (m, 1H,O-CH-O); 6,08 (m 1H,H-11).

Die anschließende Einführung des gewünschten 11β-Aryl-Substituenten erfolgt nach den z.B. in den europäischen Patentschriften EP 57 115, EP 129499, der deutschen Patentschrift DE 3438500 oder in Steroids 44, 349 [1984] beschriebenen Verfahren. Als Beispiel sei hier die Darstellung von 11β-(4-Dimethylaminophenyl-3,3-(2,2-dimethyl-trimethylen-dioxy)-13α--methyl-17β-[3-(tetrahydropyran-2-yloxy)-prop-1-inyl]-9-gonen-5α,17α-diol angeführt:

Eine Suspension von 630 mg Magnesium in 10 ml abs. THF wird mit 0,05 ml Methyliodid und anschließend trofpfenweise mit einer Lösung von 5,9 g 4-Brom-dimethylanilin in 29 ml THF versetzt, wobei die Zutropfgeschwindigkeit so gewählt wird, daß die Innentemperatur 45 °C nicht übersteigt. Nach vollständiger Auflösung der Magnesiumspäne kühlt man auf 0 °C, gibt 132 mg CuCl hinzu und rührt 15 Minuten bei 0 °C, ehe eine Lösung von 1,17 g des oben beschriebenen Propargyladdukts in 20 ml THF tropfenweise hinzugegeben wird. Danach rührt man weitere 2 Stunden bei Raumtemperatur, gießt in wäßrige $NH_3$-Lösung und extrahiert mit Ethylacetat. Nach Chromatographie des Rohprodukts an $Al_2O_3$ mit Hexan/Ethylacetat erhält man 1,24 g (84,3 %) 11β-(4-Dimethylaminophenyl)-3,3-(2,2-dimethyl-trimethylen-dioxy)-13α-methyl-17β-[3-(tetrahydropyran-2-yloxy)-prop-1-inyl]-9-gonen-5α,17α-diol als farbloses Öl, identisch mit dem in der Europäischen Patentanmeldung 847 300 62.1 (Publ.-Nr.: 0129 499) beschriebenen Produkt.

Die weitere Umwandlung zum biologisch aktiven 4,9-Gonadien-3-on-Endprodukt (d.h. gegebenenfalls Variation der C-17-Seitenkette, Wasserabspaltung unter gleichzeitiger Abspaltung vorhandener Schutzgruppen) erfolgt nach literaturb ekannten (z.B. EP 129 499, EP 57 115, Steroids 44, 349 [1984]) Verfahren.

Die Erfindung soll anhand der nachfolgenden Beispiele erläutert werden, ohne sie allerdings darauf zu beschränken.

Beispiel 1:

Eine Lösung von 4,0 g 3,3-(2,2-Dimethyl-trimethylendioxy)-5α,10α--epoxy-estr-9-en-17-on in 600 ml Hexan wird bei 25 °C 3 Stunden mit dem vollen Spektrum eines Hg-Hochdruckbrenners (Philips HPK 125) in einer Quarzglas-Tauchapparatur bestrahlt. Anschließend wird das Lösungsmittel im Wasserstrahlvakuum entfernt und der Rückstand an 700 g Aluminiumoxid (Merck, neutral, Stufe III) mit Hexan/Ethylacetat chromatographiert. Man erhält 1,96 g 3,3-(2,2-Dimethyl-trimethylendioxy)-5α, 10α-epoxy-13α-methyl-gon-9-(11)-en-17-on als farbloses Öl, $^{1}$H-NMR ($CDCl_3$): δ = 0,83 ppm (s,3H,H-18); 1,03 (breites s,6H,Ketal-$CH_3$); 5,96 (m,1H,H-11), $[\alpha]_d^{20}$ -31,9$^0$($CH_2Cl_2$,c=0,510).

Beispiel 2:

Eine Lösung von 4,0 g 3,3-(2,2-Dimethyl-trimethylendioxy)-5β,10β-epoxy-estr-9-en-17-on in 600 ml Dioxan wird 1,5 Stunden bei 20 °C unter den Bedingungen des Beispiels 1 bestrahlt. Nach Chromatographie erhält man 1,72 g 3,3-(2,2-Dimethyl-trimethylendioxy)-5β,10β-epoxy-13α-methyl-gon-9(11)--en-17-on als festen Schaum, $[\alpha]_d^{20}$ -119,8$^0$ ($CH_2Cl_2$, c=0,630), H-NMR (CDCL$_3$): δ = 0,86 ppm (s,3H,H-18); 1,05 (breites s,6H,Ketal-$CH_3$); 5,78 (m,1H,H-11).

## Patentansprüche

**1.** 13α-Alkylgon-$\Delta^{9(11)}$-en-5,10-epoxide der allgemeinen Formel I

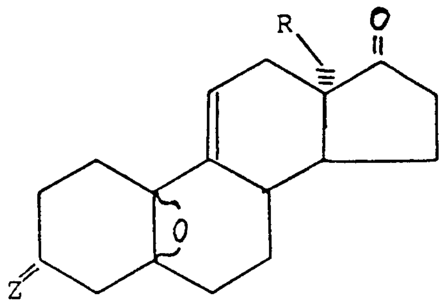

(I),

worin
R ein Wasserstoffatom, eine Methyl- oder Ethylgruppe und
Z eine in Form des Ketals oder des Thioketals blockierte Ketogruppe bedeuten.

**2.** Verfahren zur Herstellung von 13α-Alkylgon-$\Delta^{9(11)}$-en-5,10-epoxiden der allgemeinen Formel I

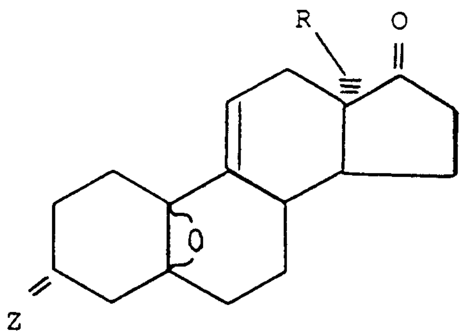

(I),

worin
R ein Wasserstoffatom, eine Methyl- oder Ethylgruppe und
Z eine in Form des Ketals oder Thioketals blockierte Ketogruppe bedeuten,
dadurch gekennzeichnet, daß man eine Verbindung der allgemeinen Formel II

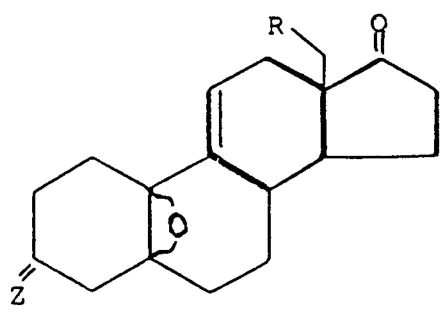

(II),

worin R und Z die in Formel I angegebene Bedeutung haben, mit ultraviolettem Licht bestrahlt.

**3.** 3,3-(2,2-Dimethyl-trimethylendioxy)-5α,10α-epoxy-13α-methyl-gon-9(11)-en-17-on
3,3-(2,2-Dimethyl-trimethylendioxy)-5β,10β-epoxy-13α-methyl-gon-9(11)-en-17-on

## Claims

1. 13α-Alkylgon-$\Delta^{9(11)}$-ene-5,10-epoxides of the general formula I

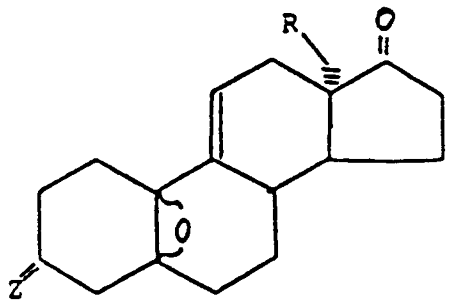

(I)

wherein
R is a hydrogen atom, a methyl group or an ethyl group, and
Z is a keto group that is blocked in the form of the ketal or the thioketal.

2. Process for the preparation of 13α-alkylgon-$\Delta^{9(11)}$-ene-5,10-epoxides of the general formula I

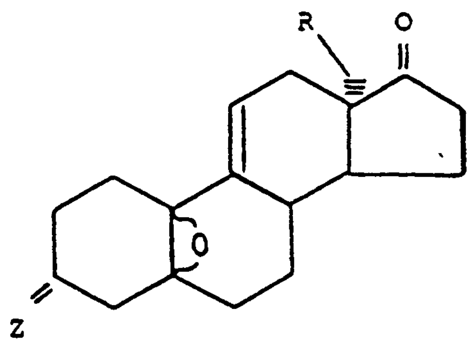

(I)

wherein
R is a hydrogen atom, a methyl group or an ethyl group, and
Z is a keto group that is blocked in the form of the ketal or the thioketal,
characterised in that a compound of the general formula II

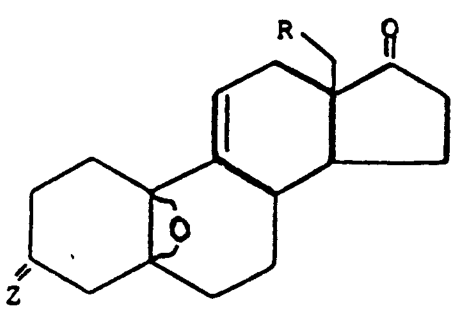

(II),

wherein R and Z have the meanings given in formula I, is irradiated with ultraviolet light.

3. 3,3-(2,2-Dimethyl-trimethylenedioxy)-5α,10α-epoxy-13α-methyl-gon-9(11)-en-17-one
3,3-(2,2-Dimethyl-trimethylenedioxy)-5β,10β-epoxy-13α-methyl-gon-9(11)-en-17-one.

## Revendications

**1.** 5,10-Epoxy-13α-alkylgon-$\Delta^{9(11)}$-ènes répondant à la formule générale I

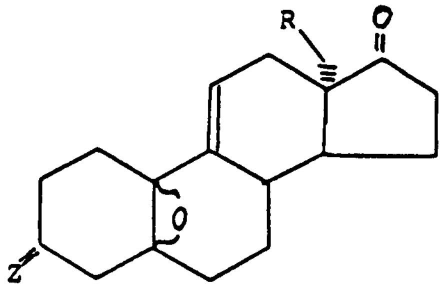

(I),

dans laquelle R représente un atome d'hydrogène ou un groupe méthyle ou éthyle, et Z représente un groupe cétonique bloqué sous la forme du cétal ou du thiocétal.

**2.** Procédé de préparation de 5,10-époxy-l3α-alkylgon-$\Delta^{9(11)}$-ènes répondant à la formule générale I

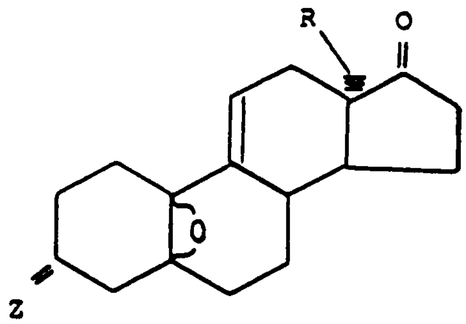

(I),

dans laquelle R représente un atome d'hydrogène ou un groupe méthyle ou éthyle et Z représente un groupe cétonique bloqué sous la forme du cétal ou du thiocétal, caractérisé en ce qu'ion irradie par de la lumière ultraviolette un composé répondant à la formule générale II

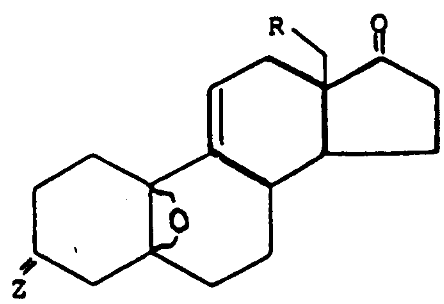

(II),

dans laquelle R et Z ont la signification indiquée dans la formule I.

**3.** 3,3-(2,2-Diméthyl-triméthylènedioxy)-5α,10α-époxy-13α-méthyl-gon-9(11)-én-17-one, 3,3-(2,2-diméthyl-triméthylènedioxy)-5β,10β-époxy-13-méthyl-gon-9(11)-én-17-one.